# EUROPÄISCHE PATENTSCHRIFT

(11) **EP 0 524 638 B1**
(45) Veröffentlichungstag und Bekanntmachung des Hinweises auf die Patenterteilung: **16.10.1996**
(21) Anmeldenummer: 92112687.6
(22) Anmeldetag: 24.07.1992
(51) Int. Cl.: C07C 31/40, C07C 29/32, C07C 29/44

(54) **Verfahren zur Herstellung von primären und sekundären fluorhaltigen Alkoholen**
Process for the preparation of fluorinated primary and secondary alcohols
Procédé de fabrication d'alcools primaires et secondaires fluorés

(30) Priorität: 26.07.1991 DE 4124807
(43) Veröffentlichungstag der Anmeldung: 27.01.1993
(73) Patentinhaber: HOECHST AKTIENGESELLSCHAFT, 65926 Frankfurt am Main (DE)
(72) Erfinder: Knaup, Wolfgang, Dr., W-8269 Burgkirchen (DE)

(56) Entgegenhaltungen:
- EP-A- 0 473 053
- US-A- 3 532 659

## Beschreibung

Die Erfindung betrifft ein Verfahren zur Herstellung von primären und sekundären fluorhaltigen Alkoholen der nachstehenden Formel 1 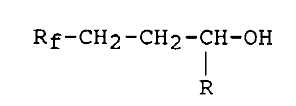 worin R_{f} ein Perfluoralkylrest mit 1 bis 20 C-Atomen ist, vorzugsweise mit 6 bis 16 C-Atomen, und R Wasserstoff ist (das sind primäre fluorhaltige Alkohole) oder ein Alkylrest mit 1 bis 5 C-Atomen ist (das sind sekundäre fluorhaltige Alkohole), durch Umsetzung von einem Perfluoralkylethylen der nachstehenden Formel 2

R_{f}-CH=CH₂

worin R_{f} die angegebene Bedeutung hat, mit einem n-C₁ bis C₆-Alkanol in Gegenwart eines Radikalinitiators.

Aus der US-Patentschrift 3,532,659 sind primäre und sekundäre fluorhaltige Alkohole der nachstehenden Formel bekannt 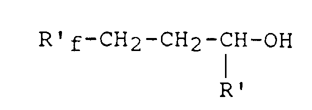 worin R'_{f} ein Perfluoralkylrest mit 5 bis 13 C-Atomen und R' Wasserstoff oder ein Alkylrest mit 1 bis 5 C-Atomen ist; vergleiche Spalte 2, Reaktionsgleichung (c) in Verbindung mit Spalte 1, Zeilen 27 bis 30 und die Beispiele 1 und 17. Die primären fluorhaltigen Alkohole werden hergestellt durch Umsetzung eines Perfluoralkylethylens der Formel R'_{f}-CH=CH₂ mit Methanol und die sekundären fluorhaltigen Alkohole durch Umsetzung eines Perfluoralkylethylens mit einem n-C₂ bis C₆-Alkanol. Die Umsetzung wird in Gegenwart eines Radikalinitiators und unter Verwendung eines mehr oder weniger großen Überschusses an Alkanol durchgeführt, und zwar in der Weise, daß die Perfluoralkylethylenverbindung, der Alkohol und der Radikalinitiator zusammengemischt werden und die Mischung auf Reaktionstemperatur erhitzt und bei dieser Temperatur bis zum Reaktionsende gehalten wird. Die in der genannten US-Patentschrift 3,532,659 beschriebene Herstellung der in Rede stehenden primären und sekundären Alkohole mit einem Perfluoralkylethylenrest erfolgt also ausschließlich in Form eines Eintopfverfahrens. Diese Verfahrensweise läßt insbesondere bezüglich Ausbeute zu wünschen übrig. So beträgt die Ausbeute im Beispiel 1 nur 80 % und im Beispiel 17 sogar nur 50 %.

Es wurde nun gefunden, daß die Umsetzung von Perfluoralkylethylenen mit n-Alkanolen in Gegenwart von Radikalinitiatoren dann zu hohen Ausbeuten an Fluoralkoholen führt, wenn man das Perfluoralkylethylen, das Alkanol und den Radikalinitiator in einem bestimmten Molverhältnis einsetzt und die Umsetzung in einer speziellen Art und Weise durchführt.

Das erfindungsgemäße Verfahren ist demnach dadurch gekennzeichnet, daS die Perfluoralkylethylenverbindung, die Alkanolverbindung und der Radikalinitiator in einem Molverhältnis von 1 zu 20 bis 50 zu 0,002 bis 0,2, Vorzugsweise 1 zu 25 bis 40 zu 0,005 bis 0,1, eingesetzt werden und die Umsetzung in der Weise durchgeführt wird, daß zunächst die Alkanolverbindung vorgelegt und auf eine Temperatur erhitzt wird, die im Bereich von 50 °C unter bis 10 °C über der 1-minütigen Halbwertszeit-Temperatur des Radikalinitiators liegt und 50 bis 230 °C, vorzugsweise 80 bis 200 °C, beträgt, und dann bei dieser Temperatur das Perfluoralkylethylen und der Radikalinitiator gleichzeitig zum Alkanol im wesentlichen kontinuierlich zudosiert werden in einer Zeit von 1 bis 10 Stunden, vorzugsweise 2 bis 8 Stunden, worauf die Mischung zur Nachreaktion noch 0,5 bis 3 Stunden bei der genannten Temperatur gehalten wird.

Das erfindungsgemäße Verfahren beruht also auf einer Kombination von im wesentlichen drei speziellen Merkmalen, das sind das angegebene Molverhältnis (1) und die angegebene Art des Inkontaktbringens von Perfluoralkylethylen, Alkohol und Radikalinitiator (2) unter Einhaltung einer bestimmten Temperatur und Dosierzeit (3).

Die beiden Reaktionskomponenten, nämlich Perfluoralkylethylen und Alkanol, und die Startradikale liefernde Verbindung werden in einem Molverhältnis von 1 zu 20 bis 50 zu 0,002 bis 0,2 eingesetzt, vorzugsweise im Molverhältnis von 1 zu 25 bis 40 zu 0,005 bis 0,1. Das Inkontaktbringen von Alkanol, Perfluoralkylethylen und Radikalstarter erfolgt in der Weise, daß die beiden letzteren Verbindungen gleichzeitig und während einer bestimmten Zeit zur Alkanolverbindung zugegeben werden, die auf eine bestimmte Temperatur erhitzt ist. Die Temperatur des Methanols, Ethanols, 1-Propanols, 1-Butanols, l-Pentanols oder 1-Hexanols wird auf einen solchen Wert eingestellt, der bis zu 50 °C unter und bis zu 10 °C über derjenigen Temperatur liegt, bei der der Radikalinitiator eine Halbwertszeit von im wesentlichen 1 Minute hat. Ein weiteres Merkmal der erfindungsgemäß einzuhaltenden Reaktionstemperatur besteht darin, daß sie nicht unter 50 °C und nicht über 230 °C, vorzugsweise nicht unter 80 °C und nicht über 200 °C, liegen soll, das sind die oben genannten Temperaturbereiche von 50 bis 230 °C, vorzugsweise 80 bis 200 °C. Im Falle des Radikalinitiators Di-tert.-butylperoxid zum Beispiel, der bei 186 °C eine Halbwertszeit von 1 Minute hat, wird also die Alkanolverbindung auf eine Temperatur von 186 °C + 10 °C/- 50 °C erhitzt, das sind 136 bis 196 °C.

In die auf die angegebene Temperatur erhitzte Alkanolverbindung werden das Perfluoralkylethylen und die Startradikale bildende Verbindung gleichzeitig und im wesentlichen kontinuierlich eingebracht, wobei zusätzlich eine bestimmte Zeit (Dosierzeit) eingehalten wird, und zwar eine Zeit von 1 bis 10 Stunden, vorzugsweise 2 bis 8 Stunden. Das gleichzeitige Zudosieren von Perfluoralkylethylen und Radikalinitiator kann zum Beispiel so erfolgen, daß man die beiden zusammenmischt und die Mischung zudosiert. Man kann die beiden Verbindungen auch getrennt voneinander gleichzeitig dosieren, zum Beispiel aus jeweils einem Gefäß heraus. Das Zugeben des Perfluoralkylethylens und des Radikalstarters zum Alkohol erfolgt mit einer solchen Dosierrate, daß eine Zeit von 1 bis 10 Stunden, vorzugsweise 2 bis 8 Stunden, verbraucht wird. Das Zugeben erfolgt ferner im wesentlichen kontinuierlich, das heißt ununterbrochen mit einer im wesentlichen konstanten Dosiermenge oder auch portionsweise, wobei aber zwischen den einzelnen Portionen keine längeren zeitlichen Intervalle (maximal etwa 5 Minuten) liegen sollten. Das Zufügen von Perfluoralkylethylen und Radikalinitiator wird also zwischen tropfenweise und mehr oder weniger leichtem Fließen liegen (im Falle der getrennten Zugabe der beiden Verbindungen wird das in einer größeren Menge einzusetzende Perfluoralkylethylen mit einer größeren Geschwindigkeit zudosiert werden als der Radikalinitiator). Nach dem Zudosieren des Perfluoralkylethylens und des Radikalinitiators zu dem auf Reaktionstemperatur erhitzten Alkohol, wird die Mischung zur Nachreaktion noch 0,5 bis 3 Stunden bei der genannten Temperatur gehalten. Das Ende der Umsetzung ist erreicht, wenn kein oder praktisch kein Perfluoralkylethylen mehr festgestellt wird. Die erfindungsgemäße Umsetzung wird ohne Verwendung eines eigenen Lösungsmittels und in einem im wesentlichen wasserfreien Medium durchgeführt. Die Anwesenheit einer geringen Wassermenge, das heißt Wasser bis zu etwa 5 Gew.-%, bezogen auf die Gewichtssumme aus Perfluoralkylethylen, Alkohol und Radikalinitiator, stört im allgemeinen nicht, so daß zum Beispiel auch technisches Methanol, Ethanol, n-Propanol, n-Butanol, n-Pentanol und n-Hexanol eingesetzt werden kann. Nachdem der Siedepunkt von zum Beispiel Methanol 65 °C und von Ethanol 78 °C ist, wird man die Umsetzung in der Regel unter Druck durchführen. Die Umsetzung kann kontinuierlich oder diskontinuierlich durchgeführt werden.

Das erfindungsgemäße Verfahren liefert die angestrebten primären und sekundären fluorhaltigen Alkohole in Form einer Lösung im überschüssig eingesetztem Alkanol. Da die Umsetzung zum angestrebten Fluoralkohol praktisch quantitativ verläuft und keine nennenswerten Nebenprodukte entstehen, ist eine Isolierung des Fluoralkohols aus der genannten Lösung oft gar nicht erforderlich. Sollte seine Isolierung gewünscht werden, wird man das überschüssige Alkanol einfach destillativ abtrennen. Dabei wird in der Regel auch gegebenenfalls noch anwesende organische Initiatorverbindung abdestilliert. Das Abtrennen von Initiatorverbindung kann zum Beispiel auch durch Waschen mit Wasser erreicht werden. Mit dem erfindungsgemäßen Verfahren werden also die angestrebten Fluoralkohole in hoher Ausbeute und hoher Reinheit erhalten.

Bezüglich der Ausgangsverbindungen für das erfindungsgemäße Verfahren sei noch folgendes gesagt:
Die Perfluoralkylgruppe R_{f} des Perfluoralkylethylens kann gerade oder verzweigt, gesättigt oder ungesättigt (mit vorzugsweise 1 bis 3 Doppelbindungen) sein, wobei gerade und gesättigt bevorzugt ist. Im Falle einer verzweigten Perfluoralkylgruppe ist die endverzweigte bevorzugt. Beim Perfluoralkylrest handelt es sich häufig um ein Gemisch von Perfluoralkyl mit der genannten Anzahl von C-Atomen, nämlich 1 bis 20 C-Atomen (CF₃ bis C₂₀F₄₁), vorzugsweise 6 bis 16 C-Atomen (C₆F₁₃ bis C₁₆F₃₃), oder mit 6 bis 12 C-Atomen (C₆F₁₃ bis C₁₂F₂₅).

Von den n-C₁ bis C₆-Alkanolen sind die ersten beiden bevorzugt, das heißt Methanol und Ethanol. Wie die angegebenen Molverhältnisse zeigen, wird die Alkanolverbindung in einem hohen Überschuß eingesetzt.

Als Radikalinitiatoren können anorganische oder organische Verbindungen eingesetzt werden. Man wird in der Regel solche Radikalinitiatoren wählen, die vorteilhafte Halbwertszeiten aufweisen und aktive Radikale liefern. Als Vertreter für anorganische Radikalinitiatoren seien Peroxosäuren, Peroxoborate, Peroxocarbonate, Peroxophosphate und Peroxodisulfate genannt. Als Vertreter für organische Radikalinitiatoren seien die organischen Peroxide und die Azoverbindungen genannt. Organische Radikalinitiatoren sind bevorzugt und davon wiederum die Peroxide, vorzugsweise Alkylhydroperoxide, Dialkylperoxide, Diacylperoxide, Peroxycarbonsäureester und Peroxycarbonsäuren. Im folgenden werden organische Peroxide im einzelnen genannt, wobei in Klammern die 1-Minuten-Halbwertszeit-Temperatur angegeben ist: tert.-Butylhydroperoxid (179 °C), Di-tert.-butylperoxid (186 °C), Diacetylperoxid (122 °C), Dilauroylperoxid (115 °C), Dibenzoylperoxid (133 °C), tert.-Butylperpivalat (112 °C) und tert.-Butylperbenzoat (163 °C).

Was die Menge an Radikalinitiator betrifft, so liegt sie - wie oben beschrieben - bei 0,002 bis 0,2 mol pro mol Perfluoralkylethylen, vorzugsweise bei 0,005 bis 0,1 mol pro mol Perfluoralkylethylen. Sie hängt insbesondere von der Halbwertszeit des Radikalinitiators ab und von der Aktivität der Radikale.

Die Erfindung wird nun an Beispielen noch näher erläutert.

### Beispiel 1

### Ansatz:

| | |
|---|---|
| 294,30 g (0,66 mol) | C₈F₁₇CH=CH₂ |
| 912,20 g (19,8 mol) | CH₃CH₂OH (wasserfrei) |
| 9,65 g (0,066 mol) | Di-tert.-butylperoxid |

Molares Verhältnis: 1 zu 30 zu 0,1.

### Durchführung:

Das Ethanol wurde in einem mit Rührer ausgestatteten Autoklaven vorgelegt, mit Stickstoff gespült und auf 160 °C erhitzt. Das Perfluoroctylethylen und das Peroxid wurden in eine Vorlage gegeben und aus der Vorlage dem erhitzten Ethanol mit Hilfe einer Dosierpumpe kontinuierlich zugeführt. Die Zulaufgeschwindigkeit war so eingestellt, daß das Perfluoroctylethylen/Peroxid-Gemisch nach 3 Stunden zudosiert war. Der Druck im Autoklaven lag bei 13 bar. Nach dem Zudosieren wurde die Mischung noch 2 Stunden lang bei der genannten Temperatur zur Nachreaktion gehalten. Zur Gewinnung des gebildeten sekundären Fluoralkohols aus dem Reaktionsgemisch wurde das überschüssige Ethanol abdestilliert und das Sumpfprodukt, das den sekundären Fluoralkohol darstellt, mehrmals mit Wasser gewaschen (diese Aufarbeitung des Reaktionsgemisches erfolgte auch in den weiteren Beispielen, wobei zum Waschen des Sumpfproduktes anstelle von Wasser auch eine schwach alkalische wäßrige Lösung eingesetzt wurde).

### Ergebnis:

Der erhaltene sekundäre Fluoralkohol C₈F₁₇CH₂CH₂CH(CH₃)OH ist bei Raumtemperatur ein farbloses, kristallines wachsartiges Produkt. Die Ausbeute betrug 95 % der Theorie.

### Beispiel 2

### Ansatz:

| | |
|---|---|
| 294,30 g (0,66 mol) | C₈F₁₇CH=CH₂ |
| 845,90 g (26,4 mol) | CH₃OH, technische Ware |
| 4,82 g (0,033 mol) | Di-tert.-butylperoxid |

Molares Verhältnis: 1 zu 40 zu 0,05.

### Durchführung:

Wie in Beispiel 1, mit dem Unterschied, daß das vorgelegte Methanol auf 170 °C erhitzt wurde. Der Druck lag bei 21 bis 22 bar. Nach dem Zudosieren des Perfluoroctylethylen/Peroxid-Gemisches (Dosierzeit 3 Stunden) wurde der Autoklaveninhalt noch 1,5 Stunden bei der genannten Temperatur zur Nachreaktion gehalten.

### Ergebnis:

Der isolierte primäre Fluoralkohol C₈F₁₇CH₂CH₂CH₂OH ist bei Raumtemperatur ein farbloses, kristallines wachsartiges Produkt. Die Ausbeute betrug 93 % der Theorie.

### Beispiel 3

### Ansatz:

| | |
|---|---|
| 294,3 g (0,66 mol) | C₈F₁₇CH=CH₃ |
| 912,2 g (19,8 mol) | CH₃CH₂OH, technische Ware |
| 5,8 g (0,033 mol) | tert.-Butylperpivalat |

Molares Verhältnis: 1 zu 30 zu 0,05.

### Durchführung:

Das Ethanol wurde in einem mit einem Rührer, Rückflußkühler, Thermometer und mit einem Tropftrichter ausgestatteten Reaktionsgefäß vorgelegt, mit Stickstoff gespült und auf 80 °C erhitzt. Das im Tropftrichter befindliche Perfluoroctylethylen/Peroxid-Gemisch wurde in einer Zeit von 5 Stunden ununterbrochen zum erhitzten Ethanol zugetropft. Nach der Zugabe wurde die Mischung noch 3 Stunden lang bei 80 °C unter Rühren zur Nachreaktion gehalten.

### Ergebnis:

Wie in Beispiel 1, mit einer Ausbeute von 93 % der Theorie.

### Beispiel 4

### Ansatz:

| | |
|---|---|
| 294,3 g (0,66 mol) | C₈F₁₇CH=CH₂ |
| 1 163,6 g (13,2 mol) | n-Amylalkohol (n-Pentanol) |
| 11,1 g (0,046 mol) | Benzoylperoxid |

Molares Verhältnis: 1 zu 20 zu 0,07.

### Durchführung:

Der Amylalkohol wurde in einem mit einem Rührer, Rückflußkühler, Thermometer und mit einem Tropftrichter und einem Pulverdosiertrichter ausgestatteten Reaktionsgefäß vorgelegt, mit Stickstoff gespült und auf 105 °C erhitzt. Im Tropftrichter befand sich das flüssige Perfluoroctylethylen und im Pulverdosiertrichter das pulverförmige Benzoylperoxid. Die Zugabe des Perfluoroctylethylens und des Benzoylperoxids aus den beiden Dosiergefäßen zum erhitzten Amylalkohol wurde so eingestellt, daß die beiden Verbindungen in einer Zeit von 2 Stunden gleichzeitig und ununterbrochen zuflossen. Nach der Zugabe wurde die Mischung noch 2 Stunden lang bei der angegebenen Temperatur zur Nachreaktion gehalten.

### Ergebnis:

Der isolierte sekundäre Fluoralkohol 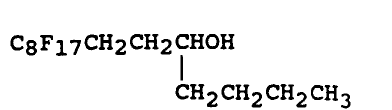 ist bei Raumtemperatur ein farbloses, kristallines wachsartiges Produkt. Die Ausbeute betrug 90 % der Theorie.

### Beispiel 5

### Ansatz:

| | |
|---|---|
| 32,4 kg (60,8 mol) | R_{f}CH=CH₂, R_{f} = C₆F₁₃ bis C₁₂F₂₅ |
| 84,0 kg (1,82 kmol) | Ethanol, technische Ware |
| 71,6 g (0,49 mol) | Di-tert.-butylperoxid |

Molares Verhältnis: 1 zu 30 zu 0,008.

### Durchführung:

Wie in Beispiel 1.

### Ergebnis:

Der erhaltene sekundäre Fluoralkohol R_{f}CH₂CH₂CH(CH₃)OH ist bei Raumtemperatur ein farbloses, kristallines wachsartiges Produkt. Die Ausbeute betrug 93 % der Theorie.

Die folgenden Vergleichsbeispiele 1 bis 5 sollen die unerwartete Wirkung der erfindungsgemäßen Verfahrensweise noch weiter beweisen.

### Vergleichsbeispiel 1

### Ansatz:

| | |
|---|---|
| 294,30 g (0,66 mol) | C₈F₁₇CH=CH₂ |
| 912,20 g (19,8 mol) | CH₃CH₂OH (wasserfrei) |
| 9,65 g (0,066 mol) | Di-tert.-butylperoxid |

Molares Verhältnis: 1 zu 30 zu 0,1.

### Durchführung:

Das Perfluoroctylethylen, das Ethanol und das Peroxid wurden in einem mit Rührer ausgestatteten Autoklaven vorgelegt und mit Stickstoff gespült. Die Reaktionsmischung wurde auf 122 °C erhitzt und 24 Stunden bei dieser Temperatur gerührt. Der Druck im Autoklaven lag bei 6 bar. Zur Gewinnung des gebildeten sekundären Fluoralkohols aus dem Reaktionsgemisch wurde überschüssiges Ethanol abdestilliert und das Sumpfprodukt, das den sekundären Fluoralkohol darstellt, mehrmals mit Wasser gewaschen.

### Ergebnis:

Der erhaltene sekundäre Fluoralkohol C₈F₁₇CH₂CH₂CH(CH₃)OH ist bei Raumtemperatur ein farbloses, kristallines wachsartiges Produkt. Die Ausbeute betrug 83 % der Theorie.

### Vergleichsbeispiel 2

### Ansatz:

| | |
|---|---|
| 294,30 g (0,66 mol) | C₈F₁₇CH=CH₂ |
| 912,20 g (19,8 mol) | CH₃CH₂OH (wasserfrei) |
| 9,65 g (0,066 mol) | Di-tert.-butylperoxid |

Molares Verhältnis: 1 zu 30 zu 0,1.

### Durchführung:

Das Perfluoroctylethylen, das Ethanol und das Peroxid wurden in einem mit Rührer ausgestatteten Autoklaven vorgelegt und mit Stickstoff gespült. Die Reaktionsmischung wurde auf 160 °C erhitzt und 24 Stunden bei dieser Temperatur gerührt. Der Druck im Autoklaven lag bei 13 bar. Zur Gewinnung des gebildeten sekundären Fluoralkohols aus dem Reaktionsgemisch wurde überschüssiges Ethanol abdestilliert und das Sumpfprodukt, das den sekundären Fluoralkohol darstellt, mehrmals mit Wasser gewaschen.

### Ergebnis:

Der erhaltene sekundäre Fluoralkohol C₈F₁₇CH₂CH₂CH(CH₃)OH ist bei Raumtemperatur ein farbloses, kristallines wachsartiges Produkt. Die Ausbeute betrug 82 % der Theorie.

### Vergleichsbeispiel 3

### Ansatz:

| | |
|---|---|
| 294,30 g (0,66 mol) | C₈F₁₇CH=CH₂ |
| 912,20 g (19,8 mol) | CH₃CH₂OH (wasserfrei) |
| 9,65 g (0,066 mol) | Di-tert.-butylperoxid |

Molares Verhältnis: 1 zu 30 zu 0,1.

### Durchführung:

Das Perfluoroctylethylen, das Ethanol und das Peroxid wurden in einem mit Rührer ausgestatteten Autoklaven vorgelegt und mit Stickstoff gespült. Die Reaktionsmischung wurde auf 160 °C erhitzt und 5 Stunden bei dieser Temperatur gerührt. Der Druck im Autoklaven lag bei 13 bar. Zur Gewinnung des gebildeten sekundären Fluoralkohols aus dem Reaktionsgemisch wurde überschüssiges Ethanol abdestilliert und das Sumpfprodukt, das den sekundären Fluoralkohol darstellt, mehrmals mit Wasser gewaschen.

### Ergebnis:

Der erhaltene sekundäre Fluoralkohol C₈F₁₇CH₂CH₂CH(CH₃)OH ist bei Raumtemperatur ein farbloses, kristallines wachsartiges Produkt. Die Ausbeute betrug 83 % der Theorie.

### Vergleichsbeispiel 4

### Ansatz:

| | |
|---|---|
| 89,2 g (0,2 mol) | C₈F₁₇CH=CH₂ |
| 114,7 g (3,58 mol) | CH₃OH, technische Ware |
| 2,92 g (0,02 mol) | Di-tert.-butylperoxid |

Molares Verhältnis: 1 zu 17,9 zu 0,1.

### Durchführung:

Das Perfluoroctylethylen, das Methanol und das Peroxid wurden in einem mit Rührer ausgestatteten Autoklaven vorgelegt und mit Stickstoff gespült. Die Reaktionsmischung wurde auf 123 °C erhitzt und 17 Stunden bei dieser Temperatur gerührt. Der Druck im Autoklaven lag bei 12 bar. Zur Gewinnung des gebildeten primären Fluoralkohols aus dem Reaktionsgemisch wurde überschüssiges Methanol abdestilliert und das Sumpfprodukt, das den primären Fluoralkohol darstellt, mehrmals mit Wasser gewaschen.

### Ergebnis:

Der erhaltene primäre Fluoralkohol C₈F₁₇CH₂CH₂CH₂OH ist bei Raumtemperatur ein farbloses, kristallines wachsartiges Produkt. Die Ausbeute betrug 59 % der Theorie.

### Vergleichsbeispiel 5

### Ansatz:

| | |
|---|---|
| 294,3 g (0,66 mol) | C₈F₁₇CH=CH₂ |
| 845,9 g (26,4 mol) | CH₃OH, technische Ware |
| 4,82 g (0,033 mol) | Di-tert.-butylperoxid |

Molares Verhältnis: 1 zu 40 zu 0,05.

### Durchführung:

Wie im Vergleichsbeispiel 4, mit dem Unterschied, daß die Reaktionsmischung 4,5 Stunden bei 170 °C gerührt wurde. Der Druck im Autoklaven lag bei 23 bar. Zur Gewinnung des gebildeten primären Fluoralkohols aus dem Reaktionsgemisch wurde überschüssiges Methanol abdestilliert und das Sumpfprodukt, das den primären Fluoralkohol darstellt, mehrmals mit Wasser gewaschen.

### Ergebnis:

Der erhaltene primäre Fluoralkohol C₈F₁₇CH₂CH₂CH₂OH ist bei Raumtemperatur ein farbloses, kristallines wachsartiges Produkt. Die Ausbeute betrug 62 % der Theorie.

## Patentansprüche

1. Verfahren zur Herstellung von primären und sekundären fluorhaltigen Alkoholen der nachstehenden Formel 1 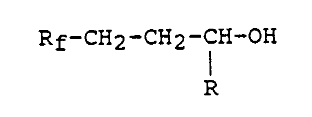 worin R_{f} ein Perfluoralkylrest mit 1 bis 20 C-Atomen ist und R Wasserstoff oder ein Alkylrest mit 1 bis 5 C-Atomen ist, durch Umsetzung von einem Perfluoralkylethylen der nachstehenden Formel 2
R_{f}-CH=CH₂
worin R_{f} die angegebene Bedeutung hat, mit einem n-C₁ bis C₆-Alkanol in Gegenwart eines Radikalinitiators, dadurch gekennzeichnet, daß die Perfluoralkylethylenverbindung, die Alkanolverbindung und der Radikalinitiator in einem Molverhältnis von 1 zu 20 bis 50 zu 0,002 bis 0,2 eingesetzt werden und die Umsetzung in der Weise durchgeführt wird, daß zunächst die Alkanolverbindung vorgelegt und auf eine Temperatur erhitzt wird, die im Bereich von 50 °C unter bis 10 °C über der 1-minütigen Halbwertszeit-Temperatur des Radikalinitiators liegt und 50 bis 230 °C beträgt, und dann bei dieser Temperatur das Perfluoralkylethylen und der Radikalinitiator gleichzeitig zum Alkanol im wesentlichen kontinuierlich zudosiert werden in einer Zeit von 1 bis 10 Stunden, worauf die Mischung zur Nachreaktion noch 0,5 bis 3 Stunden bei der genannten Temperatur gehalten wird.

2. Verfahren nach Anspruch 1, dadurch gekennzeichnet, daß die Perfluoralkylethylenverbindung, die Alkanolverbindung und der Radikalinitiator in einem Molverhältnis von 1 zu 25 bis 40 zu 0,005 bis 0,1 eingesetzt werden.

3. Verfahren nach Anspruch 1 oder 2, dadurch gekennzeichnet, daß die Alkanolverbindung auf eine Temperatur erhitzt wird, die im Bereich von 50 °C unter bis 10 °C über der 1-minütigen Halbwertszeit-Temperatur des Radikalinitiators liegt und 80 bis 200 °C beträgt.

4. Verfahren nach einem oder mehreren der Ansprüche 1 bis 3, dadurch gekennzeichnet, daß das Perfluoralkylethylen und der Radikalinitiator zum Alkanol in einer Zeit von 2 bis 8 Stunden zudosiert werden.

5. Verfahren nach Anspruch 1, dadurch gekennzeichnet, daß die Perfluoralkylethylenverbindung, die Alkanolverbindung und der Radikalinitiator in einem Molverhältnis von 1 zu 25 bis 40 zu 0,005 bis 0,1 eingesetzt werden und die Umsetzung in der Weise durchgeführt wird, daß zunächst die Alkanolverbindung vorgelegt und auf eine Temperatur erhitzt wird, die im Bereich von 50 °C unter bis 10 °C über der 1-minütigen Halbwertszeit-Temperatur des Radikalinitiators liegt und 80 bis 200 °C beträgt, und dann bei dieser Temperatur das Perfluoralkylethylen und der Radikalinitiator gleichzeitig zum Alkanol im wesentlichen kontinuierlich zudosiert werden in einer Zeit von 2 bis 8 Stunden.

6. Verfahren nach einem oder mehreren der Ansprüche 1 bis 5, dadurch gekennzeichnet, daß R_{f} ein Perfluoralkylrest mit 6 bis 16 C-Atomen ist und R Wasserstoff oder CH₃ ist.

## Claims

1. A process for the preparation of a primary or secondary fluorine-containing alcohol of the formula 1 below 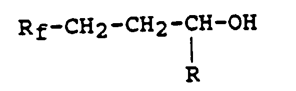 in which R_{f} is a perfluoroalkyl radical having 1 to 20 carbon atoms and R is hydrogen or an alkyl radical having 1 to 5 carbon atoms, by reaction of a perfluoroalkylethylene of the formula 2 below
R_{f}-CH=CH₂
in which R_{f} has the meaning given, with an n-C₁ to C₆-alkanol in the presence of a free radical initiator, which comprises using the perfluoroalkylethylene compound, the alkanol compound and the free radical initiator in a molar ratio of 1 to 20-50 to 0.002-0.2 and carrying out the reaction in such a manner that the alkanol compound is taken first and heated to a temperature which is in the range from 50°C below to 10°C above the one-minute halflife temperature of the free radical initiator and is 50 to 230°C and then at this temperature the perfluoroalkylethylene and the tree radical initiator are simultaneously and essentially continuously added to the alkanol in a time period of 1 to 10 hours, whereupon the mixture is held for further 0.5 to 3 hours at the temperature mentioned for continued reaction.

2. The process as claimed in claim 1, wherein the perfluoroalkylethylene compound, the alkanol compound and the free radical initiator are used in a molar ratio of 1 to 25-40 to 0.005-0.1.

3. The process as claimed in claim 1 or 2, wherein the alkanol compound is heated to a temperature which is in the range from 50°C below to 10°C above the one-minute halflife temperature of the free radical initiator and is 80 to 200°C.

4. The process as claimed in one or more of claims 1 to 3, wherein the perfluoroalkylethylene and the free radical initiator are added to the alkanol in a time period of 2 to 8 hours.

5. The process as claimed in claim 1, wherein the perfluoroalkylethylene compound, the alkanol compound and the free radical initiator are used in a molar ratio of 1 to 25-40 to 0.005-0.1 and the reaction is carried out in such a manner that the alkanol compound is taken first and heated to a temperature which is in the range from 50°C below to 10°C above the one-minute halflife temperature of the free radical initiator and is 80 to 200°C and then at this temperature the perfluoroalkylethylene and the free radical initiator are simultaneously and essentially continuously added to the alkanol in a time period of 2 to 8 hours.

6. The process as claimed in one or more of claims 1 to 5, wherein R_{f} is a perfluoroalkyl radical having 6 to 16 carbon atoms and R is hydrogen or CH₃.

## Revendications

1. Procédé pour préparer des alcools primaires et secondaires fluorés ayant la formule 1 ci-après 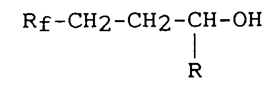 dans laquelle R_{f} est un résidu perfluoralkyle ayant de 1 à 20 atomes de carbone, et R est un hydrogène ou un radical alkyle ayant de 1 à 5 atomes de carbone, par réaction d'un perfluoralkyléthylène ayant la formule 2 ci-après :
R_{f}-CH=CH₂
dans laquelle R_{f} a la signification donnée, avec un n-alcanol en C₁ à C₆ en présence d'un amorceur radicalaire, caractérisé en ce que le perfluoralkyléthylène, l'alcanol et l'amorceur radicalaire sont utilisés selon une proportion en moles de 1:20 à 50:0,002 à 0,2, et que l'on met en oeuvre la réaction en partant d'abord de l'alcanol et en le chauffant à une température qui est comprise entre 50°C en-dessous et 10°C au-dessus de la température de demi-vie à 1 minute de l'amorceur radicalaire, et qui est de 50 à 230°C, puis, à cette température, on ajoute simultanément à l'alcool, d'une manière essentiellement continue, le perfluoralkyléthylène et l'amorceur radicalaire, en un laps de temps de 1 à 10 minutes, le mélange, pour assurer une réaction subséquente, étant encore maintenu pendant 0,5 à 3 heures à la température indiquée.

2. Procédé selon la revendication 1, caractérisé en ce que le perfluoralkyléthylène, l'alcanol et l'amorceur radicalaire sont utilisés selon une proportion en moles de 1:25 à 40:0,005 à 0,1.

3. Procédé selon la revendication 1 ou 2, caractérisé en ce que l'alcanol est chauffé à une température comprise entre 50°C en-dessous et 10°C au-dessus de la température de demi-vie à 1 minute de l'amorceur radicalaire, et qui est de 80 à 200°C.

4. Procédé selon l'une ou plusieurs des revendications 1 à 3, caractérisé en ce que le perfluoralkyléthylène et l'amorceur radicalaire sont ajoutés à l'alcanol en un laps de temps de 2 à 8 heures.

5. Procédé selon la revendication 1, caractérisé en ce que le perfluoralkyléthylène, l'alcanol et l'amorceur radicalaire sont utilisés en une proportion en moles de 1:25 à 40:0,005 à 0,1, et on met en oeuvre la réaction en partant d'abord de l'alcanol et en le chauffant à une température comprise entre 50°C en-dessous et 10°C au-dessus de la température de demi-vie à 1 minute de l'amorceur radicalaire et qui est de 80 à 200°C, puis, à cette température, on ajoute essentiellement en continu en un laps de temps de 2 à 8 heures le perfluoralkyléthylène et l'amorceur radicalaire, en même temps que l'alcanol.

6. Procédé selon l'une ou plusieurs des revendications 1 à 5, caractérisé en ce que R_{f} est un résidu perfluoralkyle ayant de 6 à 16 atomes de carbone, et R est un hydrogène ou CH₃.
